(19) Europäisches Patentamt / European Patent Office / Office européen des brevets

(11) **EP 1 053 785 B1**

(12) **FASCICULE DE BREVET EUROPEEN**

(45) Date de publication et mention
de la délivrance du brevet:
**18.07.2007 Bulletin 2007/29**

(51) Int Cl.:
*B01J 21/06* (2006.01)     *B01J 37/03* (2006.01)
*B01J 27/053* (2006.01)     *C07C 5/27* (2006.01)
*C10G 49/02* (2006.01)

(21) Numéro de dépôt: **00401272.0**

(22) Date de dépôt: **10.05.2000**

(54) **Support catalytique à base d'oxyde d'un métal du groupe IVB de la classification périodique des éléments, sa préparation et ses utilisations**

Ein auf einem metalloxid der Gruppe IVB basierende Katalysatorträger, seine Herstellung und seine Verwendungen

Catalytic support based on metal oxide of group IVB, its preparation and uses thereof

(84) Etats contractants désignés:
**AT BE CH CY DE DK ES FI FR GB GR IE IT LI LU MC NL PT SE**

(30) Priorité: **18.05.1999 FR 9906283**

(43) Date de publication de la demande:
**22.11.2000 Bulletin 2000/47**

(73) Titulaire: **TotalFinaElf France**
**92800 Puteaux (FR)**

(72) Inventeurs:
• **Da Silva, Pedro**
**76600 Le Havre (FR)**
• **Bisson, Marc**
**76700 Gainneville (FR)**

• **Milan, Alain**
**76290 Fontaine La Mallet (FR)**
• **Decker, Sébastien**
**76170 Triquerville (FR)**
• **Denayer, Joeri**
**1750 Sint-Martens-Lennik (BE)**

(74) Mandataire: **Jolly, Jean-Pierre et al**
**Cabinet Jolly**
**54, rue de Clichy**
**75009 Paris (FR)**

(56) Documents cités:
**EP-A- 0 174 836          EP-A- 0 520 543**
**EP-A- 0 644 158          WO-A-97/18892**

**Description**

La présente invention concerne un support catalytique particulièrement approprié pour la fabrication de catalyseurs de traitement d'hydrocarbures, et qui comprend en particulier au moins un oxyde d'un métal du groupe IVB de la Classification Périodique des Eléments, auquel est incorporée une faible quantité de silice. L'invention comprend également des méthodes privilégiées pour la préparation d'un tel support. L'invention comprend enfin des catalyseurs élaborés à partir d'un tel support, ainsi que les utilisations de ces catalyseurs dans des réactions de traitement d'hydrocarbures, telles que, par exemple, les réactions d'hydrodésulfuration, d'hydrodéazotation, d'hydrogénation, d'hydroisomérisation....

De manière connue en soi, l'industrie pétrolière a recours à de nombreux procédés dont le but est de transformer sélectivement certains composés présents dans les coupes pétrolières, afin d'obtenir des produits dont les propriétés conviennent à l'usage recherché. Ces procédés font généralement appel à un ou plusieurs catalyseurs solides, lesquels doivent être spécifiquement adaptés à la transformation chimique que l'on souhaite effectuer, ainsi qu'aux exigences liées à la mise en oeuvre du procédé.

Bon nombre de ces réactions se font en présence d'une quantité plus ou moins importante d'hydrogène. C'est le cas, par exemple, des réactions d'hydrodésulfuration et d'hydrodéazotation, qui visent à éliminer de certaines coupes pétrolières les composés indésirables que sont respectivement les hydrocarbures soufrés et azotés. C'est également le cas des réactions intervenant dans le procédé d'isomérisation des paraffines, lequel s'applique essentiellement aux essences légères et permet de transformer les paraffines linéaires en paraffines ramifiées, dont l'indice d'octane est plus élevé.

Dans la plupart de ces procédés de traitement de coupes pétrolières, les catalyseurs traditionnellement employés à l'heure actuelle sont essentiellement constitués d'un support d'alumine de haute porosité, sur lequel est déposée une phase active, qui correspond aux sites actifs du catalyseur. Cette phase active comprend souvent une fonction favorisant les transferts d'hydrogène (en particulier un métal du groupe VIII de la Classification Périodique des Eléments), généralement associé à un autre composé, spécifique de l'activité recherchée, c'est à dire de la réaction à catalyser.

Toutefois, de manière générale, l'activité des catalyseurs traditionnels s'avère désormais insuffisante, compte tenu des exigences croissantes en terme de rendements des procédés industriels. Par exemple, il est aujourd'hui fondamental d'accroître l'efficacité des procédés d'hydrodésulfuration, en réponse à des normes environnementales de plus en plus sévères sur les teneurs maximales des carburants en composés soufrés.

C'est pourquoi de nombreuses recherches ont été entreprises afin d'élaborer de nouveaux catalyseurs, plus actifs, qui permettraient de répondre à ces objectifs sans avoir à modifier de manière importante les unités existantes, ce qui permettrait d'éviter des investissements coûteux.

De manière connue en soi, la nature et les propriétés de son support influent considérablement sur l'activité d'un catalyseur, et les chercheurs ont entre autres essayé de remplacer les supports traditionnels à base d'alumine par de nouveaux supports, aptes à conférer aux catalyseurs une activité supérieure. En particulier, les oxydes des métaux du groupe IVB de la Classification Périodique des Eléments, tels que par exemple la zircone, se sont très vite avérés représenter des candidats potentiels relativement intéressants.

Déjà, en 1970, le brevet US N° 3 686 095 mentionne la possibilité théorique de remplacer l'alumine par de la zircone ou de la magnésie. Toutefois, ce brevet se borne à décrire un catalyseur d'hydrodésulfuration comprenant une phase active (métal hydrogénant associé à un métal du groupe VI) déposée sur un support de très haute porosité constitué d'alumine mélangée à de la silice, c'est à dire un catalyseur à base d'alumine de type tout-à-fait traditionnel. S'il mentionne, de manière théorique, l'emploi possible d'oxydes alternatifs, il ne décrit absolument pas comment l'on peut effectivement préparer des supports constitués de tels oxydes, présentant une porosité suffisante pour servir de base à la préparation de catalyseurs industriels.

En effet, si les oxydes du type de la zircone présentent effectivement des propriétés intéressantes, ils se sont jusqu'à présent avérés peu appropriés à la fabrication de supports de catalyseurs industriels, dans la mesure où l'obtention d'une porosité suffisante se traduit par la perte des propriétés spécifiques apportées par ces oxydes. Aussi de nombreuses tentatives ont-elles été faites, visant à améliorer les supports à base de tels oxydes, dans le but d'en faire des supports catalytiques utilisables dans l'industrie.

Ainsi, le brevet US N° 5 021 385 propose un catalyseur comprenant un support de porosité élevée constitué de zircone et d'oxyde de titane coprécipités, sur lequel sont déposés de l'oxyde de molybdène (2 à 30% en poids), de l'oxyde de nickel ou de cobalt (1 à 10% en poids), et éventuellement du phosphore.

Le brevet FR N° 2 661 171 décrit un procédé de synthèse d'une zircone stabilisée de haute surface spécifique, destinée à servir de support de catalyseur d'hydrotraitement. La porosité élevée de ce catalyseur est obtenue grâce à l'imprégnation de la zircone amorphe, avant calcination, par une solution d'un élément stabilisant choisi parmi l'yttrium, le nickel, l'aluminium, le titane, le phosphore.

Le brevet US N° 5 262 373 préconise la méthode dite des sels fondus pour préparer un support à base de zircone, d'alumine, de silice, ou d'oxyde de titane, seuls ou en mélange ; le support préféré contient de la zircone, seule ou mélangée à de l'alumine, et est destiné, après dépôt de nickel et de molybdène, à servir de catalyseur d'hydrotraitement.

Le brevet FR N° 2 709 432 revendique un catalyseur contenant un support de surface spécifique supérieure ou égale à 150 m$^2$/g, constitué de 60 à 99% en poids de zircone et de 1 à 40% en poids d'oxyde d'au moins un métal choisi dans le groupe constitué par les métaux des groupes V, VI, VII, les métaux nobles tels que le ruthénium, l'osmium, le rhodium, l'iridium, l'uranium, les éléments phosphore, arsenic et soufre. Ce support, essentiellement destiné à des catalyseurs d'hydrotraitement d'hydrocarbures, est également préparé par la méthode des sels fondus.

D'une manière générale, les catalyseurs proposés dans l'art antérieur comme alternatives aux catalyseurs traditionnels sur support d'alumine se sont avérés peu satisfaisants, en raison de leur niveau d'activité insuffisant, c'est à dire inférieur à celui des catalyseurs traditionnels. En effet, l'obtention d'une porosité élevée du support se fait au détriment du supplément d'activité apporté par l'oxyde métallique alternatif à l'alumine. En d'autres termes, lorsque l'on souhaite obtenir un support suffisamment poreux (ce qui est indispensable pour garantir une bonne accessibilité des sites actifs aux molécules à convertir), on perd les propriétés catalytiques spécifiques qui font tout l'intérêt de ces oxydes métalliques. C'est pourquoi de tels catalyseurs ne sont, à l'heure actuelle, généralement pas employés dans des réacteurs industriels.

WO-A-9 718 892 décrit un catalyseur acide et la méthode pour l'obtenir.

Poursuivant ses recherches dans le domaine des catalyseurs à base de zircone ou autres oxydes équivalents, la Demanderesse a cherché à agir sur les propriétés de ces oxydes. Ce faisant, elle a découvert que, de manière surprenante, la zircone ou l'oxyde de titane peuvent devenir des matériaux de choix permettant de réaliser d'excellents supports catalytiques, à condition de présenter une structure de type cristallin et d'être dopés de manière appropriée avec une faible quantité de silice. Il devient en effet possible de contrôler rigoureusement toutes les caractéristiques de porosité, sans perdre pour autant l'activité spécifique apportée par ces oxydes alternatifs. La Demanderesse a donc pu élaborer des catalyseurs industriels à partir d'une matrice de porosité élevée, essentiellement constituée d'oxydes métalliques du groupe IVB (dont la zircone et d'oxyde de titane), lesquels catalyseurs se sont avérés présenter un niveau d'activité supérieur par rapport aux catalyseurs de l'art antérieur.

Elle a également découvert une méthode de préparation originale, qui permet d'obtenir de tels supports en contrôlant leur porosité de manière à obtenir les structures actives désirées.

Ainsi, la Demanderesse a mis au point un support catalytique comprenant une quantité substantielle d'au moins un oxyde de métal du groupe IVB de la Classification Périodique des Eléments, auquel est incorporée de la silice, caractérisé en ce que le rapport massique entre la quantité d'oxyde métallique du groupe IVB et la quantité de silice qu'il contient est compris entre 5 et 70, en ce l'oxyde métallique du groupe IVB est sous forme cristalline, et en ce que la surface spécifique du support est supérieure ou égale à 160 m$^2$/g.

Ici et dans ce qui suit, les caractéristiques de porosité (surface spécifique, volume de pores et rayon de pores moyen) sont citées en référence à la méthode de détermination dite B.E.T (Brunauer, Emmett, Teller), bien connue de l'homme du métier.

Les caractéristiques de structure et de texture du support selon l'invention ont été optimisées en agissant sur son mode de fabrication, en particulier en incorporant à l'oxyde métallique du groupe IVB une faible quantité de silice, selon un rapport massique déterminé, et en mettant en oeuvre des traitements thermiques appropriés, de manière à contrôler précisément la cristallinité de l'oxyde métallique du groupe IVB. La Demanderesse est ainsi parvenue à contrôler les propriétés de ces oxydes, ce qui lui a permis de sélectionner les formules les plus actives, objet de la présente invention.

Comparé à l'art antérieur, le support selon l'invention permet de réaliser des catalyseurs plus actifs, plus appropriés à une utilisation en réacteur industriel. En effet, il présente une porosité élevée, ce qui garantit l'accessibilité des sites actifs aux réactifs, tout en préservant l'activité spécifique supplémentaire apportée par le remplacement de l'alumine par un oxyde métallique du groupe IVB. En d'autres termes, les catalyseurs élaborés à partir de tels supports présentent des sites à la fois plus actifs et plus accessibles, ce qui contribue à leurs excellentes performances.

De plus, les propriétés du support selon l'invention sont stables à haute température. En particulier, sa porosité élevée ne se dégrade ni dans les conditions opératoires d'un réacteur industriel, ni lorsqu'il est soumis à un traitement thermique sévère, contrairement à bon nombre des catalyseurs antérieurs, dont le support n'est pas essentiellement l'alumine. Cette stabilité constitue un avantage indéniable du support selon l'invention. En particulier, les catalyseurs fabriqués à partir de ce support seront parfaitement régénérables, dans des conditions classiques de régénération (par exemple, dans le cas des catalyseurs de traitement d'hydrocarbures, par oxydation ménagée à température croissante, de manière à provoquer la combustion progressive du coke déposé à la surface du catalyseur).

Par ailleurs, comparé aux supports catalytiques traditionnels à base d'alumine, le support selon l'invention permet de préparer des catalyseurs présentant une activité accrue dans des conditions réactionnelles similaires. En particulier, les résultats de tests de cinétique d'adsorption et de désorption d'azote sur ce support montrent que celui-ci présente une forme de pores particulière, vraisemblablement analogue à une cavité cylindrique, ce qui augmente les vitesse d'entrée et de sortie des réactifs dans les pores par rapport aux supports à base d'alumine, qui présentent des pores dont la forme peut être assimilée à celle d'une bouteille d'encre, c'est-à-dire avec une restriction de la section d'entrée/sortie des pores, d'où un risque d'encombrement de ceux-ci. Ce phénomène contribue à expliquer le gain d'activité constaté pour les catalyseurs fabriqués à partir du support selon l'invention.

Enfin, ces catalyseurs peuvent tout-à-fait être employés dans des réacteurs de type classique, où il suffit de les substituer, totalement ou partiellement, aux catalyseurs sur support d'alumine. Ceci permet, de manière simple et peu coûteuse, d'accroître sensiblement les performances des procédés considérés, en particulier les procédés de traitement d'hydrocarbures.

Parallèlement, la Demanderesse a mis au point une méthode de préparation, qui permet de maîtriser la structure et la texture des supports catalytiques à base d'oxyde métallique du groupe IVB, et de préparer ainsi un support tel que décrit ci-dessus.

Enfin, la Demanderesse a utilisé ce support pour préparer un certain nombre de catalyseurs destinés essentiellement au traitement d'hydrocarbures.

L'invention concerne donc également la méthode de préparation du support, tel que décrit dans le libellé de la revendication independante 20 ainsi que tout catalyseur préparé à partir de ce support. Ces aspects seront exposés plus en détail dans la description et les exemples qui suivent.

Dans la présente invention, le support catalytique joue un rôle fondamental, dans la mesure où l'activité supérieure des catalyseurs d'hydrotraitement obtenus in fine, c'est-à-dire après dépôt des phases actives, dépend largement des propriétés intrinsèques du support. Ces propriétés doivent donc être contrôlées de manière particulièrement rigoureuse.

Le support catalytique selon l'invention contient une quantité substantielle d'au moins un oxyde d'au moins un métal du groupe IVB, lequel oxyde doit être impérativement présent sous forme cristalline, c'est à dire qu'il ne doit pas se trouver à l'état amorphe. De préférence, l'oxyde métallique du groupe IVB se trouve sous une forme cristalline de type tétragonal. La détermination de la structure cristalline dudit oxyde métallique est réalisée, de manière connue en soi, par diffraction des rayons X.

La texture du support est par ailleurs contrôlée grâce à l'incorporation, dans ledit oxyde métallique du groupe IVB, d'une faible quantité de silice, avec un rapport pondéral donné : la teneur en poids du mélange en oxyde métallique du groupe IVB est comprise entre 5 et 70 fois sa teneur en silice, ce qui correspond à une teneur en silice comprise entre 1,5% à 16 % en poids dans un mélange qui ne contiendrait que ces deux oxydes. De préférence, le rapport massique entre l'oxyde métallique du groupe IVB et la silice est compris entre 8 et 30. Par silice, on désigne tout oxyde de silicium.

L'oxyde métallique du groupe IVB que renferme le support est préférentiellement choisi parmi la zircone, l'oxyde de titane ou un mélange de ces deux oxydes. De préférence, ledit oxyde métallique du groupe IVB est la zircone. Par zircone, on désigne le dioxyde de zirconium.

De préférence, la surface spécifique dudit support est supérieure ou égale à 180 m$^2$/g. Il présente avantageusement un volume de pores supérieur ou égal à 0,25 cm$^3$/g, de préférence compris entre 0,30 et 0,60 cm$^3$/g inclus. Par ailleurs, son rayon de pores moyen est avantageusement supérieur ou égal à 20 Å, de préférence compris entre 20 et 120 Å inclus.

Le support catalytique selon l'invention comprend essentiellement le mélange décrit ci-dessus, constitué d'oxyde métallique du groupe IVB (en particulier de la zircone, de l'oxyde de titane ou un mélange des deux) sous forme cristallisée, dopé à la silice.

Il peut comprendre en outre une quantité variable d'un autre oxyde métallique réfractaire usuellement employé dans l'industrie et que, de manière connue en soi, l'homme du métier peut juger bon d'incorporer à diverses fins, par exemple afin d'obtenir ou renforcer certaines propriétés, afin de faciliter la préparation ou la mise en forme du catalyseur, ou encore afin de diminuer les coûts de fabrication du catalyseur en substituant une partie du support par des matières premières moins coûteuses ou plus largement disponibles que les oxydes métalliques du groupe IVB.

En particulier, le support selon l'invention peut avantageusement comprendre un agent liant qui a été incorporé afin de faciliter sa mise en forme. Un tel liant peut être constitué de tout oxyde minéral réfractaire habituellement employé dans ce but, et peut être notamment choisi dans le groupe constitué par les alumines, les silices, les silice-alumines, les alumino-silicates, les argiles et les mélanges de ces composés. De préférence, ledit liant est constitué d'alumine. Il peut avantageusement être incorporé en quantité comprise entre 0,1 et 30 % en poids par rapport au poids total du support.

Le support catalytique selon l'invention constitue une base appropriée pour la fabrication de toutes sortes de catalyseurs dits supportés, c'est-à-dire comprenant, déposés sur ledit support, un ou plusieurs composés jouant le rôle de phase active. Des composés très divers peuvent ainsi être déposés sur ce support, en fonction du type d'activité recherché, c'est à dire de la réaction à catalyser.

Lorsque le catalyseur est destiné à être employé dans un procédé de traitement de coupes hydrocarbonées, ladite phase active comprend avantageusement au moins un métal ou un composé d'un métal du groupe VIII de la Classification Périodique des Eléments. Les métaux ou composés métalliques de cette famille sont en effet particulièrement connus pour faciliter les réactions impliquant des transferts d'hydrogène (ce qui est souvent le cas des réactions intervenant dans les traitements d'hydrocarbures).

Selon un premier mode de réalisation, la phase active est constituée d'un ou plusieurs métaux ou composés de métaux du groupe VIII de la Classification Périodique des Eléments. Ledit métal du groupe VIII peut par exemple être le nickel, à une teneur comprise entre 15 et 30% en poids par rapport au poids total du catalyseur. Il peut également, à titre d'exemple non limitatif, être choisi parmi le platine et le palladium, et être présent à une teneur comprise entre 0,1 et 2% en poids par rapport au poids total du catalyseur.

Les catalyseurs selon ce premier mode de réalisation sont particulièrement appropriés pour une utilisation dans des réactions d'hydrogénation d'hydrocarbures.

D'autre part, un métal ou un composé métallique du groupe VIII peut être associé à un ou plusieurs composés supplémentaires, métalliques ou non, choisis pour leur activité spécifique dans la réaction à catalyser.

Ainsi, selon un deuxième mode de réalisation, ladite phase active est constituée d'un ou plusieurs métaux ou composés de métaux du groupe VIII de la Classification Périodique des Eléments, associé à au moins un composé de type acide. Les métaux du groupe VIII sont alors préférentiellement choisis parmi le platine, le palladium, le ruthénium, le rhodium, l'osmium, l'iridium, et le nickel. Le platine, seul ou associé au palladium, est particulièrement préféré. Ces métaux sont avantageusement présents à une teneur totale comprise entre 0,1 et 2 % en poids par rapport au poids total du catalyseur. Le composé de type acide peut comprendre par exemple des ions sulfate, des ions tungstate, à une teneur variable selon le degré d'acidité recherché.

Les catalyseurs décrits selon ce deuxième mode de réalisation sont particulièrement appropriés pour une utilisation dans des réactions nécessitant l'emploi d'un catalyseur de type acide ou superacide, telles que notamment des réactions d'isomérisation de paraffines ou d'oléfines, des réactions de décyclisation d'hydrocarbures naphténiques, des réactions d'alkylation, des réactions d'oligomérisation, des réactions de déshydratation d'hydrocarbures, des réactions d'hydrogénation de coupes pétrolières.

Ces catalyseurs sont également particulièrement avantageux pour des réactions d'hydrodéaromatisation et/ou de désulfuration profonde de coupes pétrolières, dont notamment des gazoles.

Enfin, ces catalyseurs se sont avérés particulièrement performants dans des procédés de traitement de coupes hydrocarbonées contenant une quantité substantielle de paraffines à longue chaîne (à plus de 7 atomes de carbone), linéaires ou peu ramifiées, telles que par exemple des paraffines issues d'une synthèse du type Fischer-Tropsch (synthèse d'hydrocarbures à partir du mélange $CO+H_2$). La transformation de ces paraffines par hydrocraquage et/ou par hydroisomérisation est souvent nécessaire afin d'obtenir soit des " grands " produits (essences, naphta, distillats moyens), soit des spécialités (paraffines, lubrifiants haut de gamme). Les conditions opératoires doivent alors être ajustées en fonction de la réaction que l'on souhaite favoriser (hydrocraquage ou hydroisomérisation), et du niveau de conversion souhaité. Elles peuvent être avantageusement les suivantes : une température comprise entre 20°C et 400°C (de préférence entre 150°C et 300°C), une pression de préférence comprise entre $5.10^5$ et $200.10^5$ Pa, un rapport moléculaire hydrogène/hydrocarbures $H_2$/HC compris entre 1 et 20 (de préférence entre 5 et 15).

Selon un troisième mode de réalisation, qui constitue le mode préféré de réalisation de l'invention, la phase active comprend au moins un métal ou un composé d'un métal du groupe VIII de la Classification Périodique des Eléments, associé à au moins un métal ou un composé d'un métal du groupe VIB de la Classification Périodique des Eléments. Ces deux types de métaux sont avantageusement déposés sur le support sous forme d'oxydes de métaux, lesquels sont ensuite transformés, totalement ou partiellement, en sulfures correspondants (sulfures de métaux) au cours d'une étape d'activation précédant l'utilisation du catalyseur.

Ledit métal du groupe VIII est de préférence le cobalt et/ou le nickel. Le catalyseur selon l'invention comprend de préférence de 2 à 10 % en poids d'oxyde métallique correspondant.

Ledit métal du groupe VIB est de préférence le molybdène et/ou le tungstène. Le catalyseur selon l'invention comprend de préférence de 10 à 25 % en poids d'oxyde métallique correspondant.

Les catalyseurs selon ce troisième mode de réalisation sont particulièrement appropriés pour une utilisation dans des procédés d'hydrotraitement tels que, notamment, les procédés d'hydrodésulfuration, d'hydrodéazotation, d'hydrodémétallation ou d'hydrogénation de coupes pétrolières telles que des essences, des gazoles, des distillats sous vide, des résidus atmosphériques ou des résidus sous vide.

Les conditions de mise en oeuvre de ces procédés d'hydrotraitement seront optimisées en fonction du type d'hydrotraitement envisagé, mais également en fonction de la nature de la charge à traiter et du taux de traitement que l'on souhaite atteindre. Les conditions opératoires des procédés d'hydrotraitement ne sont pas substantiellement modifiées, en présence d'un catalyseur conforme à l'invention, par rapport aux conditions usuelles en présence d'un catalyseur traditionnel sur support d'alumine. Ces conditions sont généralement les suivantes : une température comprise entre 260 et 400°C, une pression totale comprise entre 20 et $200.10^5$ Pa (entre 20 et 200 bars), un rapport volumique hydrogène / hydrocarbures compris entre 50 et 2000 normaux-litres / litre, une vitesse volumique horaire de la charge de préférence comprise entre 0,2 et 10 $h^{-1}$.

Les catalyseurs décrits ci-dessus, bien que constituant des modes préférés de réalisation de l'invention, ne sauraient en limiter la portée. L'homme du métier peut en effet réaliser une grande variété de catalyseurs supportés à partir du support catalytique objet de l'invention, en déposant sur celui-ci les phases actives appropriées.

Les catalyseurs selon l'invention sont de type solide. Ils peuvent se présenter sous toutes les formes auxquelles l'homme du métier recourt habituellement pour la mise en oeuvre de catalyseurs solides, et notamment sous la forme de particules telles que des billes, des extrudés, des pastilles. Ils ont une densité apparente de remplissage généralement comprise entre 0,7 et 1,2 g/cm$^3$.

La méthode employée pour la préparation du support selon l'invention doit permettre de maîtriser rigoureusement ses propriétés structurelles et texturales, ceci afin d'obtenir un support répondant aux caractéristiques de l'invention et constituant une base appropriée pour la préparation de catalyseurs hautement actifs, répondant aux exigences des procédés industriels. Les caracteristiques essentielles du procédé de préparation sont décrites dans le libellé de la revendication independante 20.

La Demanderesse a mis au point un procédé privilégié pour la préparation d'un tel support catalytique. Ce procédé comprend essentiellement les étapes suivantes :

a) à partir d'une solution d'au moins un sel d'au moins un métal du groupe IVB, précipitation du ou des oxydes métalliques correspondants,
b) mûrissement à reflux du précipité obtenu,
c) addition de silice,
d) mûrissement du mélange, sous agitation,
e) lavage, filtration puis séchage du solide obtenu,
f) mise en forme du solide,
g) calcination du solide.

L'étape (a) de précipitation du ou des oxyde(s) métallique(s) du groupe IVB peut avantageusement être effectuée par addition d'une solution basique à une solution (par exemple aqueuse) dans laquelle sont dissous des sels métalliques appropriés. La solution basique employée peut être toute solution permettant, par augmentation du pH, de réaliser la précipitation d'un oxyde hydraté à partir d'une solution d'un sel précurseur dudit oxyde. Il pourra s'agir par exemple d'une solution d'ammoniaque ou de toute autre base connue de l'homme du métier.

Les sels des métaux du groupe IVB susceptibles d'être employés en vue de l'obtention du précipité d'oxyde métallique du groupe IVB sont bien connus de l'homme du métier. Lorsque l'on souhaite obtenir un support à base de zircone, on emploie un sel de zirconium qui peut par exemple être choisi dans le groupe constitué par les nitrate, chlorure, acétate, formiate, oxalate de zirconium ou de zirconyle, ainsi que les alcoolates de zirconium. Lorsque l'on souhaite obtenir un support à base de d'oxyde de titane, on emploie un sel de titane, qui peut par exemple être choisi parmi les chlorures de titane, les alcoolates de titane.

L'étape (b) de mûrissement s'effectue en portant à reflux la solution dans laquelle est précipité le gel d'oxyde métallique du groupe IVB pendant une durée suffisante, avantageusement comprise entre 1 et 50 heures et de préférence entre 12 et 36 heures.

Concernant l'étape (c), la silice est ajoutée au gel d'oxyde métallique du groupe IVB. Cet ajout peut se faire de diverses manières, par exemple par addition directe de silice, ou par addition d'une solution d'un sel de silicium et précipitation in situ. Dans le premier cas, on ajoute au gel d'oxyde métallique du groupe IVB une solution contenant un gel de silice colloïdale. Dans le second cas, on ajoute au gel d'oxyde métallique du groupe IVB une solution d'un sel de silicium (tel que l'orthosilicate d'éthyle, le silicate de sodium) et l'on provoque la précipitation de silice par addition d'un acide.

L'étape (d) de mûrissement s'effectue en maintenant une agitation vigoureuse du mélange des gels de silice et d'oxyde métallique du groupe IVB, pendant une durée avantageusement comprise entre 1 et 100 heures. Ce mûrissement peut être effectué à reflux ou à température ambiante. Il est de préférence effectué à reflux lorsque l'oxyde métallique du groupe IVB est l'oxyde de titane.

L'étape (e) correspond à une filtration de la suspension résultante, afin de récupérer le gel d'oxydes. Celui-ci est alors lavé, puis séché à température modérée (de préférence autour de 100°C), par exemple à l'étuve.

L'étape (f) de mise en forme du solide permet d'agglomérer le solide en poudre, de manière à former des particules (billes, extrudés ou pastilles par exemple), afin d'obtenir un support apte à la préparation de catalyseurs utilisables directement dans un réacteur industriel. Pour faciliter cette opération, il peut être nécessaire d'ajouter un liant (xérogel d'alumine ou tout autre liant industriel) à la poudre, puis de malaxer le mélange ainsi obtenu, avant de procéder à la mise en forme proprement dite par extrusion, " oil drop ", méthode du drageoir ou toute autre méthode connue pour la mise en forme des catalyseurs industriels.

Le support ainsi obtenu est soumis à une étape (g) de calcination, qui doit avoir lieu à une température suffisamment élevée, avantageusement comprise entre 550 et 850°C, pendant une durée qui est de préférence comprise entre 2 et 6 heures. Cette étape est fondamentale, puisqu'elle aboutit à la cristallisation de l'oxyde métallique du groupe IVB. Cette calcination peut avantageusement être effectuée sous débit d'air contrôlé.

La préparation de catalyseurs à partir du support selon l'invention se fait de manière classique, par dépôt d'au moins une phase active sur ledit support, selon des méthodes bien connues de l'homme du métier. Bien entendu, la méthode employée dépendra largement de la nature des phases actives à déposer sur le support.

Pour le dépôt de composés métalliques, on peut avantageusement procéder par imprégnation du support au moyen de solutions de composés des métaux à déposer, suivie de séchage. Par exemple, si l'on souhaite déposer du platine sur le support, on peut procéder par imprégnation au moyen d'une solution d'un composé du platine pouvant être choisi dans le groupe constitué par l'acide chloroplatinique et les composés complexes du platine.

Lorsque la phase active du catalyseur à préparer comprend différents composés, ceux-ci peuvent être, selon leur nature, déposés soit successivement, soit simultanément.

En particulier, pour la préparation d'un catalyseur comprenant au moins un métal (ou composé métallique) du groupe VIII associé à au moins un métal (ou composé métallique) du groupe VIB, il est préférable de déposer simultanément ces différents composants, par imprégnation par une solution d'un mélange des sels correspondants, suivie de séchage.

Généralement, un tel dépôt de phase active est suivi d' une étape de calcination du catalyseur

Par ailleurs, pour certains types de phases actives il peut être avantageux de procéder au dépôt avant l'achèvement complet de la préparation du support, et notamment avant l'étape g) de calcination du support. C'est le cas par exemple pour des phases actives constituées de sulfates, de tungstates, ou d'autres composés analogues, dont le greffage à la surface du support est meilleur lorsqu'il est réalisé avant la première calcination (étape g). Pour de telles phases actives, il peut donc être préférable d'insérer, dans la séquence de préparation du support, au moins une étape de dépôt de cette phase active. Avantageusement, ladite étape de dépôt est insérée entre les étapes e) et f), ou entre les étapes f) et g).

Enfin, avant de pouvoir utiliser le catalyseur en réacteur industriel, il sera généralement nécessaire de le soumettre à une étape finale d'activation (souvent effectuée in situ), dont les conditions, connues de l'homme du métier, dépendent essentiellement du type de procédé considéré.

Par exemple, dans les procédés d'hydrodésulfuration, d'hydrodéazotation et d'hydrodémétallation de charges d'hydrocarbures, après avoir chargé le catalyseur dans le réacteur, on procède à une étape préalable d'activation in situ, qui consiste à présulfurer les sites actifs du catalyseur par des méthodes connues en soi : en général, après mise sous pression d'hydrogène entre 50 et 200°C, on monte la température jusqu'à environ 300 à 400°C, en faisant passer sur le catalyseur des composés susceptibles de générer du soufre, tels que des mélanges d'hydrogène et de sulfure d'hydrogène, des mercaptans, des disulfures ou du sulfure de carbone, ou encore un gazole contenant du soufre.

Les méthodes de préparation décrites ci-dessus ne sont que des suggestions pour la réalisation des supports catalytiques et des catalyseurs conformes à l'invention. Bien entendu, celles-ci ne sauraient être considérées comme limitatives. Par ailleurs, l'homme du métier saura parfaitement, si nécessaire, adapter les méthodes décrites par d'autres opérations supplémentaires bien connues, telles que par exemple des étapes de lavages aux solvants, des séchages, des calcinations, l'incorporation d'autres oxydes réfractaires usuels.

Les exemples suivants sont uniquement destinés à illustrer l'invention, et ne sauraient par conséquent avoir un quelconque caractère limitatif.

EXEMPLES

Exemple 1

A. Préparation d'échantillons de support catalytique selon l'invention

Support S1

Cet essai concerne la préparation d'un support catalytique à base de zircone, à laquelle est incorporé 4 % en poids de silice.

On prépare une solution de zirconium en dissolvant 49,7 g de nitrate de zirconyle $ZrO(NO_3)_2.6H_2O$ (Aldrich) dans 497 ml d'eau distillée sous agitation. On précipite le gel d'hydroxyde de zirconium, par addition de 76 ml d'une solution d'ammoniaque à 28 %, sous forte agitation. Le pH final est de 10. On laisse mûrir le gel, toujours sous agitation, pendant 48 heures à reflux. On le refroidit à température ambiante. Son pH est alors de 9,2. On ajoute 20 ml de la solution d'ammoniaque à 28 %, sous forte agitation, pour ajuster le pH à 10.

On ajoute 34,5 ml d'eau, sous agitation, à 2,76 g de silice colloïdale AS40 (Dupont de Nemours). Puis on verse cette solution de silice sur le gel de zircone, et on laisse le mélange mûrir, sous forte agitation, pendant deux heures.

Après filtration et lavage jusqu'à pH 7 (redispersion dans 350 ml d'eau), on sèche le gel pendant une nuit à 120 °C. On obtient 21,58 g de solide.

La mise en forme est réalisée dans une seringue extrudeuse (1 mm de diamètre), après broyage et malaxage de l'ensemble du solide, avec 5,40 g de xérogel d'alumine de type Pural SB (Condea) et 23,5 ml d'eau distillé. Après séchage pendant une nuit à 120 °C, on calcine les extrudés à 640 °C pendant 4 heures. La quantité de support ainsi obtenue est de l'ordre de 20 g.

Support S2

Cet essai concerne la préparation d'un support catalytique à base de zircone, à laquelle est incorporé 5 % en poids de silice.

On part, comme pour le support S1, de 49,7 g de $ZrO(NO_3)_2.6H_2O$. On précipite le gel de zircone de la même manière, mais le reflux ne dure que 36 heures.

On refroidit le gel à température ambiante. Le pH de celui-ci est alors de 9,3. On ajoute 20 ml de la solution d'ammoniaque à 28 %, sous agitation, pour ajuster le pH à 10.

On ajoute 43 ml d'eau, sous agitation, à 3,48 g de silice AS40. On verse cette silice sur le gel de zircone, et on laisse mûrir le mélange sous forte agitation, pendant 2 heures. Après filtration et lavage jusqu'à pH 7 (redispersion dans 350 ml d'eau), on sèche le gel pendant une nuit à 120 °C. On obtient 21,91 g de solide.

La mise en forme est réalisée comme pour le support S1, mais avec 5,48 g d'alumine Pural SB et 22,9 ml d'eau. Les extrudés sont également séchés une nuit à 120 °C et calcinés 4 heures à 640 °C. La quantité de support ainsi obtenue est de l'ordre de 20 g.

Support S3

Cet essai concerne la préparation d'un support catalytique à base de zircone, à laquelle est incorporé 7,5 % en poids de silice.

On part, comme pour le support S1, de 49,7 g de $ZrO(NO_3)_2 \ 6H_2O$ et on précipite le gel de zircone de la même manière. Le reflux dure 36 heures. Le pH du gel, à température ambiante, est de 9,4 et on ajoute 13 ml de solution d'ammoniaque à 28 % pour ajouter celui-ci à 10.

On ajoute 68 ml d'eau, sous agitation, à 5,37 g de silice AS40, puis on procède de la même manière que pour le support S1. La quantité de matière récupérée, après séchage, est de 22,75 g. La mise en forme est réalisée de la même manière que pour le support S1, mais avec 5,69 g d'alumine Pural SB et 23,9 ml d'eau. Les extrudés sont séchés une nuit, à 120 °C, et calcinés 4 heures, à 640 °C. La quantité de support ainsi obtenue est de l'ordre de 20 g.

Support S4

Cet essai concerne la préparation d'un support catalytique à base d'oxyde de titane, auquel est incorporé 5 % en poids de silice.

On prépare une solution de titane en ajoutant 180 ml d'eau, sous agitation, à 231,4 g d'une solution de $TiCl_3$ (Prolabo) à 15 % poids dans l'eau. Cette solution est refroidie dans de la glace.

On précipite le gel d'hydroxyde de titane par addition, goutte à goutte, de 150 ml d'une solution d'ammoniaque à 28 %, sous forte agitation. Le pH final est de 10.

On laisse mûrir ce gel, toujours sous agitation, pendant 6 heures à reflux. On le refroidit à température ambiante. Son pH est alors de 9,7. On ajoute 7 ml d'une solution d'ammoniaque à 28 %, sous agitation, pour ajuster le pH à 10.

On ajoute 30 ml d'eau, sous agitation, à 2,37g de silice AS40. On verse cette silice sur le gel d'hydroxyde de titane, sous forte agitation, et on laisse mûrir ce gel, toujours sous forte agitation, pendant 24 heures à reflux. Après filtration et lavage jusqu'à élimination complète des ions chlorures (Test $AgNO_3$ négatif), on sèche le gel pendant une nuit à 120 °C. On obtient 22,43 g de solide.

La mise en forme est réalisée, de la même manière que pour le support S1, mais avec 5,86 g d'alumine Pural SB et 21,7 ml d'eau. Les extrudés sont séchés une nuit à 120 °C, et calcinés 4 heures à 575 °C. La quantité de support ainsi obtenue est de l'ordre de 20 g.

B. Propriétés des échantillons de support catalytique

Le Tableau I ci-après illustre les propriétés des échantillons de support catalytique obtenus selon les méthodes de préparation exposées ci-dessus.

Dans ces quatre échantillons, l'oxyde métallique du groupe IVB présente une structure cristalline de type tétragonale. Dans cet exemple et dans ceux qui suivent, la structure de la zircone a été déterminée par diffraction des rayons X.

Le support S5 est un support d'alumine gamma de type classique. Il correspond aux supports catalytiques traditionnellement employés et est présenté ici à titre de référence représentative de l'art antérieur.

Dans le Tableau I, S, Vp et Rp désignent respectivement la surface spécifique, le volume de pores et le rayon de pores moyen du catalyseur.

Dans cet exemple et dans ceux qui suivent, ces caractéristiques ont été déterminées au moyen de la méthode dite B.E.T. (Brunauer, Emmett, Teller), par adsorption d'azote, telle qu'elle est décrite dans l'ouvrage de S. Lowell intitulé " Powder Surface Area and Porosity ", Society of Petroleum Engineers Advanced Technology Series (1984). Plus précisément, la surface spécifique et le volume de pores ont été déterminés par la méthode B.E.T par adsorption d'azote en utilisant l'appareil ASAP 2400 commercialisé par Micromeritics. La surface spécifique S est déduite de la transformée linéaire B.E.T. à cinq points (aux pressions relatives P/Po = 0,045 ; 0,08 ; 0,15 ; 0,25 et 0,33), le volume de pores Vp est déterminé d'après la quantité d'azote adsorbé à P/Po = 0,985 et le rayon de pores moyen Rp est calculé par la formule Rp = 2Vp / S.

Avant la détermination de ces caractéristiques, l'échantillon a été soumis à un prétraitement par dégazage sous vide primaire à 250 °C pendant au moins 8 heures.

La forme des pores a été déduite des isothermes d'adsoption / désorption d'azote. Pour les échantillons de support selon l'invention (S1 à S4), les cinétiques d'adsorption et de désorption de l'azote sont quasi identiques, tandis que, dans le cas du support à base d'alumine (S5), la cinétique de désorption est nettement moindre par rapport à la cinétique d'adsorption. Cette différence peut notamment s'expliquer par la forme des pores, analogue à celle d'une cavité cylindrique, dans le premier cas, et à celle d'une bouteille d'encre, dans le second cas.

Tableau I

| Support | Oxyde gr. IVB / silice (rapport massique) | S $(m^2/g)$ | Vp $(cm^3/g)$ | Rp $(10^{-10}m)$ | Cristallinité | Formes des pores |
|---------|-------------------------------------------|-------------|---------------|------------------|---------------|------------------|
| $S_1$ | 24 | 199 | 0,34 | 37 | tétragonale | cavité cylindrique |
| $S_2$ | 19 | 203 | 0,36 | 37 | tétragonale | cavité cylindrique |
| $S_3$ | 12 | 199 | 0,37 | 37 | tétragonale | cavité cylindrique |
| $S_4$ | 19 | 211 | 0,38 | 32 | tétragonale | cavité cylindrique |
| $S_5$ | / | 271 | 0,75 | 39 | gamma | "bouteille d'encre" |

C. Préparation d'échantillons de catalyseur

Les supports S1 à S5 décrits ci-dessus ont été utilisés afin de préparer des échantillons de catalyseur d'hydrotraitement d'hydrocarbures, par dépôt à leur surface d'un métal du groupe VIB (le molybdène), sous forme d'oxyde, et d'un métal du groupe VIII (le cobalt), sous forme d'oxyde.

Les échantillons de catalyseurs C1 à C5, respectivement préparés à partir des supports S1 à S5, possèdent tous la composition suivante, illustrée dans le Tableau II.

Tableau II

| | |
|---|---|
| Quantité de support | 81,5 % en poids |
| Teneur en $MoO_3$ | 15,4 % en poids |
| Teneur en CoO | 3,1 % en poids |

Ces échantillons de catalyseur ont été préparés de la manière suivante :

a) Préparation de solutions d'imprégnation

Solution de molybdène :

- dissolution de 3,10g d'heptamolydate d'ammonium dans une solution aqueuse constituée de 7 à 14 $cm^3$ d'eau distillée et de 2,1g d'éthylène diamine.

Solution de cobalt :

- dissolution de 1,95 g de nitrate de cobalt dans 7 à 14 cm$^3$ d'eau distillée.

Ces solutions sont utilisées pour l'imprégnation de 20 cm$^3$ de support et sont toujours utilisées immédiatement après leur préparation. Le volume d'eau utilisé a été ajusté en fonction de la porosité du support à imprégner. Pour les échantillons présentés ici , ce volume était toujours compris entre 7 et 14 cm$^3$.

b) Imprégnation des échantillons de support catalytique

L'imprégnation du support préalablement séché à l'étuve à 120°C est réalisée dans un ballon animé d'un mouvement rotatif et sous flux permanent d'azote. L'ajout de la solution de molybdène sur le support en mouvement est assuré par une pompe à débit constant de 30 cm$^3$ / heure. A l'issue de cette opération le support imprégné est maintenu dans le ballon pendant une heure, mais avec une vitesse de rotation plus lente. Ce support est ensuite séché pendant deux heures à l'étuve à 120 °C, avant d'être replacé dans le ballon rotatif pour l'imprégnation par la solution de cobalt, qui est réalisée de la même manière que pour le molybdène.

c) Séchage et calcination des échantillons de catalyseur

A la suite d'un séchage de 16 heures à l'étuve à 120 °C, chaque échantillon est calciné à une température de 500 °C sous flux contrôle d'air (50 litres/heure). La vitesse de montée en température est de 1,2°C/minute et le palier à 500 °C est maintenu pendant une durée de 4 heures.

Le Tableau III ci-dessous illustre les caractéristiques des échantillons de catalyseur ainsi obtenus.

Tableau III

| Catalyseur | S (m$^2$/g) | Vp (cm$^3$/g) | Rp (10$^{-10}$m) |
|---|---|---|---|
| C1 | 171 | 0,31 | 34 |
| C2 | 162 | 0,30 | 35 |
| C3 | 158 | 0,30 | 37 |
| C4 | 155 | 0,30 | 30 |
| C5 | 238 | 0,55 | 37 |

D. Activité des échantillons de catalyseur

L'activité des échantillons C1 à C5 a été déterminée dans le procédé d'hydrodésulfuration d'une coupe pétrolière de type gazole. Les tests ont été réalisés dans un réacteur pilote, dans des conditions identiques à celles des procédés industriels.

a) Chargement et activation du catalyseur

Pour chaque test, 20 cm$^3$ de l'échantillon de catalyseur concerné ont été dilués dans 47 cm$^3$ de carborumdum et l'ensemble a été chargé dans le réacteur. Puis le catalyseur a été activé in situ, par présulfuration des oxydes de cobalt et de molybdène présents à sa surface, en faisant passer sur le catalyseur une charge de présulfuration composée de gazole straight run à 1,4 % en poids de soufre, additivée à 1 % en poids de diméthyldisulfure, dans les conditions suivantes :

- température : 360°C
- pression totale : 30.10$^5$ Pa
- vvh(volume de charge par unité de volume de catalyseur et par heure) : 3,0 h$^{-1}$
- rapport hydrogène/hydrocarbure (H$_2$/HC): 150Nl/l (normaux-litres / litre)

b) Conditions du test

Les échantillons de catalyseur ainsi activés ont été utilisés pour désulfurer une charge d'hydrocarbures qui est une coupe pétrolière classique, constituée d'un mélange isovolumique de gazole straight run (50 % en volume) et de LCO, (de l'anglais "Light Cycle Oil", gazole issu d'un procédé de craquage catalytique (50% en volume). Cette charge contient 1,72 % en poids de soufre.

Les conditions opératoires retenues pour tester les échantillons de catalyseur sont les suivantes :

- pression totale :       $30.10^5$ Pa
- vitesse spatiale horaire (v.v.h.) :       3,0 $h^{-1}$
- rapport $H_2$/HC :       150 Nl/l

A chaque fois, l'échantillon de catalyseur a été soumis à une première étape de stabilisation d'une durée minimale de 24 heures, pendant laquelle on fait passer la charge sur le catalyseur dans les conditions opératoires du test, à une température de 360 °C.

Après cette étape de stabilisation, on procède au test proprement dit, par analyse des effluents issus du pilote à différentes températures de réaction (trois températures ont été testées : 340 °C; 360°C; 380°C).

c) Activité des échantillons de catalyseur

Le Tableau IV ci-après illustre les résultats des tests de désulfuration.

Tableau IV

| Catalyseur | Densité de chargement (g/cm³) | Teneur en soufre des effluents (% en poids) | | |
|:---:|:---:|:---:|:---:|:---:|
| | | A 340°C | A 360°C | A 380°C |
| C1 | 0,96 | 0,186 | 0,075 | 0,031 |
| C2 | 0,95 | 0,175 | 0,067 | 0,028 |
| C3 | 0,97 | 0,184 | 0,101 | 0,034 |
| C4 | 0,81 | 0,215 | 0,092 | 0,045 |
| C5 | 0,75 | 0,218 | 0,122 | 0,055 |

Les résultats ci-dessus montrent que les catalyseurs conformes à l'invention (à savoir les catalyseurs C1, C2, C3, C4) permettent d'obtenir des effluents dont la teneur en soufre est nettement inférieure à celle obtenue avec le catalyseur C5, qui est un catalyseur classique à base d'alumine.

Autrement dit, dans les conditions du test, qui correspondent aux conditions classiques d'hydrodésulfuration en réacteur industriel, les catalyseurs élaborés à partir du support selon l'invention présentent une excellente activité, puisqu'ils permettent de désulfurer la charge de manière plus poussée que les catalyseurs traditionnels.

Le Tableau V ci-après permet de comparer de manière plus précise les performances des échantillons de catalyseur selon l'invention (C1 à C4) aux performances du catalyseur traditionnel C5. Ce tableau correspond aux résultats obtenus pour les tests de désulfuration effectués à une température de 360 °C.

Tableau V

| Catalyseur | Taux de désulfuration de la charge (% en poids) | ΔT (en °C) | RVA |
|:---:|:---:|:---:|:---:|
| C1 | 95,6 % | -13 | 142 |
| C2 | 96,1 % | -15 | 154 |
| C3 | 94,1 % | -6 | 115 |
| C4 | 94,6 % | -8 | 123 |
| C5 | 92,9 % | 0 | 100 |

Le paramètre ΔT, calculé pour les catalyseurs C1 à C4 en prenant comme référence le catalyseur traditionnel C5, correspond au gain de température qui serait réalisé avec ces catalyseurs C1 à C4, en opérant à taux de désulfuration identique.

Le paramètre RVA, (de l'anglais "Relative Volume Activity") permet de comparer les activités des échantillons de catalyseur, à volume d'échantillon égal, à l'activité de l'échantillon de référence C5.

$$RVA = \frac{(So/S1)^{n-1}_{échantillon} - 1}{(So/S1)^{n-1}_{référence} - 1}$$

avec :

    So = taux de soufre initial (en poids)
    S1 = taux de soufre des effluents (en poids)
    n = ordre de la réaction = 1,6

Les résultats ci-dessus illustrent les performances accrues des catalyseurs selon l'invention.

D'une part, dans des conditions opératoires identiques, ils permettent une désulfuration plus complète de la charge.

D'autre part, le gain d'activité apporté par ces catalyseurs peut être utilisé afin d'opérer dans des conditions plus douces. En effet, le paramètre ΔT montre que les catalyseurs selon l'invention (C1 à C4) permettent d'obtenir un même taux de désulfuration en opérant à température plus basse que les catalyseurs traditionnels (C5). Les avantages liés à une température de réaction plus modérée sont bien connus de l'homme du métier : en particulier, une température de réaction trop élevée, d'une part, est inconciliable avec les contraintes liées à la métallurgie du réacteur et, d'autre part, risque de désactiver prématurément le catalyseur, par dépôt de coke à sa surface.

Enfin, le paramètre RVA montre que, pour obtenir un taux de désulfuration identique, la quantité de catalyseur à utiliser est moins importante, si l'on utilise un catalyseur selon l'invention, que si l'on utilise un catalyseur traditionnel.

Il ressort des exemples exposés ci-dessus que les supports selon la présente invention permettent de réaliser d'excellents catalyseurs d'hydrotraitement, qui peuvent très avantageusement remplacer les catalyseurs traditionnels dans les procédés industriels.

Exemple 2

Cet exemple illustre la préparation d'un catalyseur de type acide, par dépôt sur un support selon l'invention d'une phase active constituée d'un métal du groupe VIII de la Classification Périodique des Eléments, associé à des ions tungstate.

A. Préparation et caractérisation du support

On prépare une solution de zirconium en dissolvant 52,5 g de nitrate de zirconyle $ZrO(NO_3)_2.6H_2O$ (Aldrich) dans 525 ml d'eau distillée. La solution est agitée à chaud pour parfaire la dissolution du sel, puis refroidie à température ambiante. On précipite alors le gel d'hydroxyde de zirconium, par addition de 80 ml d'une solution d'ammoniaque à 28 %, sous agitation. On laisse mûrir le gel, toujours sous agitation, pendant 36 heures à reflux. On le refroidit à température ambiante, et on ajoute 17 ml de la solution d'ammoniaque à 28 %, sous forte agitation, pour ajuster le pH à 10.

On ajoute 45 ml d'eau, sous agitation, à 3,6 g de silice colloïdale AS40 (Dupont de Nemours). Puis on mélange cette solution de silice avec la solution de gel de zircone, en ajoutant 10 ml de la solution d'ammoniaque à 28 %, de manière à maintenir un pH de 10. On laisse mûrir le mélange pendant 36 heures à reflux, sous agitation. Après filtration et lavage jusqu'à pH 7, on sèche le gel à 120°C.

On prélève un échantillon de ce gel, que l'on calcine pendant 3 heures à 750°C, de manière à pouvoir, après refroidissement, caractériser l'échantillon de support ainsi préparé. Cet échantillon présente les caractéristiques suivantes :

-    Rapport massique zircone/silice :19,
-    Surface spécifique : 210 $m^2/g$,

- Volume de pores : 0,47 cm$^3$/g,
- Structure de la zircone : structure cristalline, de type tétragonal.

B. Préparation et caractérisation du catalyseur

24,3 g du gel de zircone-silice séché sont mélangés à une solution de 150 ml l'eau distillée dans laquelle ont été dissous 5,4 g de métatungstate d'ammonium. Le solvant est évaporé à l'évaporateur rotatif, puis le solide ainsi obtenu est séché pendant une nuit à 120°C.

La mise en forme est alors effectuée dans une seringue extrudeuse (1 mm de diamètre), après broyage et malaxage de 23,25 g du solide avec 5,81 g de xérogel d'alumine de type Pural SB (Condea) et 22 ml d'eau distillée. Après séchage pendant une nuit à 120 °C, on calcine les extrudés à 750 °C pendant 3 heures.

On procède ensuite au dépôt du platine, de manière à obtenir une teneur en platine de 0,5% en poids par rapport au poids total du catalyseur. Ce dépôt est effectué de manière classique par imprégnation, c'est à dire par mise en contact des extrudés avec une solution aqueuse de $Pt(NH_3)_4Cl_2.H_2O$. Après évaporation de la phase aqueuse à l'aide d'un évaporateur rotatif, le solide est séché pendant une nuit à 120°C, et enfin calciné pendant 4 heures, à 480°C.

Le catalyseur ainsi obtenu présente une surface spécifique de 179 m$^2$/g, et un volume de pores de 0,41 cm$^3$/g.

C. Activité du catalyseur

L'activité du catalyseur a été étudiée dans une réaction d'hydroconversion de paraffines longues, dans un réacteur pilote et dans des conditions analogues à celles des procédés industriels.

10 g de catalyseur ont été placés dans le réacteur, puis le catalyseur a été activé in situ, par réduction sous un débit de 61/h d'hydrogène à pression atmosphérique et à une température de 300°C pendant 16 heures.

Le catalyseur ainsi activé a été utilisé pour isomériser une charge d'hydrocarbures constituée à 100% de normal-dodécane. Cette dernière est séchée au maximum sur zéolithe 3A, avant d'être mélangée à de l'hydrogène et mise en contact avec le catalyseur.

Les conditions opératoires sont les suivantes :

- pression totale :           30.10$^5$ Pa,
- vitesse spatiale horaire en poids (p.p.h.) :          1,0 h$^{-1}$,
- rapport molaire $H_2$/HC :          3.

Plusieurs tests ont été réalisés, en faisant varier la température. Les résultats obtenus figurent dans le Tableau VI ci-après :

Tableau VI

| Température (°C) | Conversion (%) | Rendement i-C12 (%) | Rendement C5-C11 (%) | Sélectivité i-C12 (%) | Sélectivité C5-C11 (%) | Sélectivité C1-C4 (%) |
|---|---|---|---|---|---|---|
| 210 | 28,5 | 25,1 | 0,9 | 87,9 | 3,1 | 9,0 |
| 220 | 43,1 | 35,9 | 3,1 | 83,3 | 7,3 | 9,4 |
| 230 | 62,4 | 36,8 | 15,8 | 59,0 | 25,3 | 15,7 |
| 240 | 78,5 | 28,7 | 35,2 | 36,6 | 44,8 | 18,6 |
| 250 | 97,3 | 12,3 | 73,1 | 12,6 | 75,1 | 12,3 |

Comme le montre le tableau ci-dessus, la conversion est substantielle dès 210 °C, ce qui indique une bonne activité du catalyseur.

Deux réactions se produisent essentiellement :

- hydroisomérisation du normal-dodécane en iso-dodécane (n-C12 $\rightarrow$ iC12)
- hydrocraquage du normal-dodécane en hydrocarbures plus légers, avec à chaque fois une excellente sélectivité en favaur d'hydrocarbures intermédiaires contenant de 5 à 11 atomes de carbone ; il s'agit essentiellement d'heptanes et d'octanes (C7 et C8) ramifiés, qui sont des produits recherchés, car facilement valorisables

Les résultats ci-dessus montrent qu'aux températures inférieures à 230 °C, le catalyseur selon l'invention permet d'hydroisomériser le normal-dodécane en iso-dodécane avec une excellente sélectivité (supérieure à 80%). A des températures plus élevées, la sélectivité de cette réaction diminue, au profit des réactions d'hydrocraquage. Le catalyseur selon l'invention présente alors une excellente activité pour le craquage du normal-dodécane, avec une très bonne sélectivité en faveur d'hydrocarbures intermédiaires valorisables (C5 à C11). A l'inverse, la formation d'hydrocarbures légers (C1 à C4), peu recherchés, reste très limitée.

Exemple 3

Cet exemple qui suit illustre la préparation d'un catalyseur de type acide, par dépôt sur un support selon l'invention d'une phase active constituée de deux métaux du groupe VIII de la Classification Périodique des Eléments, associés à des ions tungstate.

A. Préparation et caractérisation du catalyseur :

On prépare un catalyseur supporté selon la méthode décrite dans l'Exemple 2. La seule différence concerne l'étape finale de dépôt du métal du groupe VIII : en plus du platine, on dépose, également par imprégnation, du palladium. Ces deux métaux sont déposés de manière à ce que le catalyseur présente une teneur finale de 0,7% en poids de chacun d'entre eux (0,7% de Pt et 0,7% de Pd)

Immédiatement avant le dépôt des ions tungstate, un échantillon du gel de zircone-silice a été prélevé et calciné pendant 3 heures à 750°C, de manière à pouvoir, après refroidissement, caractériser l'échantillon de support ainsi préparé. Cet échantillon présente les caractéristiques suivantes :

- Rapport massique zircone/silice : 19,
- Surface spécifique : 222 $m^2$/g,
- Volume de pores : 0,41 $cm^3$/g,
- Rayon de pores moyen : $37.10^{-10}$m,
- Structure de la zircone : structure cristalline, de type tétragonal.

A. Activité du catalyseur :

L'activité du catalyseur a été étudiée dans une réaction d'hydrodéaromatisation d'une coupe d'hydrocarbures riche en composés aromatiques lourds, dans un réacteur pilote et dans des conditions analogues à celles des procédés industriels.

20 ml de catalyseur ont été placés dans le réacteur, puis le catalyseur a été activé in situ de la manière suivante : calcination sous un flux d'air sec à 440°C pendant 4 heures, refroidissement à température ambiante, purge à l'azote, puis réduction sous débit d'hydrogène à 300°C pendant 8 heures.

Le catalyseur ainsi activé a été utilisé pour hydrogéner une charge d'hydrocarbures constituée de 90,4% en poids de normal-dodécane et de 9,6% en poids de 2-méthyl-naphtalène (hydrocarbure comportant deux noyaux aromatiques).

La charge est séchée au maximum sur tamis moléculaire (3Å), avant d'être mélangée à de l'hydrogène et mise en contact avec le catalyseur.

Les conditions opératoires sont les suivantes :

- pression totale :      $50.10^5$ Pa,
- température d'entrée du réacteur :      235°C,
- vitesse spatiale horaire en volume (v.v.h.) :      1 $h^{-1}$,
- rapport volumique $H_2$/HC :      500 Nl/l.

Les Tableau VII ci-après présente les résultats de ce test, en termes de composition (% en poids) de la charge de départ et des effluents :

Tableau VII

| | Charge | Effluents |
|---|---|---|
| Teneur en hydrocarbures saturés non cycliques | 90,4 | 96,1 |
| Teneur en hydrocarbures bicycliques, dont : <br> - aromatiques, dont: | 9,6 <br> 9,6 | 3,9 <br> 2,1 |

(suite)

|  | Charge | Effluents |
|---|---|---|
| * 2-méthyl-naphtalène | 9,6 | 0 |
| *méthyl-tétraline | 0 | 2,1 |
| - saturés (méthyl-décaline) | 0 | 1,8 |

Les résultats ci-dessus illustrent les excellentes performances du catalyseur selon l'invention pour l'hydrodéaromatisation de coupes pétrolières. En effet, le 2-méthyl-naphtalène est intégralement converti.

Deux types de réaction se produisent essentiellement :

- hydrogénation du 2-méthyl-naphtalène en méthyl-tétraline (un seul noyau aromatique) et méthyl-décaline (pas de noyau aromatique), avec un taux de conversion de 100%
- ouverture des hydrocarbures cycliques résultant de l'hydrogénation du 2-méthyl-naphtalène, avec un taux de conversion de 59,4%.

Il est à noter que, parallèlement à ces réactions d'hydrodéaromatisation, une partie substantielle du normal-dodécane est hydro-isomérisée en iso-dodécane, ou hydrocraquée en paraffines intermédiaires ramifiées comprenant 5 à 11 atomes de carbone.

La formation d'hydrocabures légers indésirables (1 à 4 atomes de carbone) reste très minoritaire (1,8% en poids dans les effluents).

Le catalyseur de l'Exemple 3 présente donc une excellente activité pour l'hydrodéaromatisation de coupes d'hydrocarbures, puisqu'il permet non seulement d'hydrogéner, mais également de décycliser, les composés aromatiques présents, tout en limitant les phénomènes de craquage en hydrocarbures légers indésirables. Il s'avère par exemple particulièrement intéressant pour des procédés d'hydrotraitement de coupes gazoles, dans lesquels il a pour effet non seulement de diminuer la teneur en composés aromatiques indésirables et d'augmenter l'indice de cétane, mais également d'améliorer les propriétés d'écoulement à froid (grâce à l'isomérisation des normal-paraffines en iso-paraffines).

En conclusion, les Exemples 1 à 3 ci-dessus illustrent, de manière non limitative, la variété des catalyseurs susceptibles d'être préparés à partir du support selon l'invention, en déposant sur ce support une ou plusieurs phases actives , adaptées à la réaction que l'on souhaite catalyser. Ils illustrent également l'excellent degré d'activité des catalyseurs susceptibles d'être préparés à partir de ce support.

**Revendications**

1. Support catalytique essentiellement constitué d'au moins un oxyde de métal du groupe IVB de la Classification Périodique des Eléments, auquel est incorporée de la silice, **caractérisé en ce que** le rapport massique entre la quantité d'oxyde métallique du groupe IVB et la quantité de silice qu'il contient est compris entre 5 et 70, **en ce que** l'oxyde métallique du groupe IVB est sous forme cristalline, et **en ce que** la surface spécifique du support est supérieure ou égale à 160 $m^2$/g.

2. Support catalytique selon la revendication 1, **caractérisé en ce que** le rapport massique entre l'oxyde métallique du groupe IVB et la silice est compris entre 8 et 30.

3. Support catalytique selon l'une quelconque des revendications précédentes, **caractérisé en ce que** l'oxyde métallique du groupe IVB est choisi parmi la zircone, l'oxyde de titane ou un mélange de ces deux oxydes.

4. Support catalytique selon la revendication 3, **caractérisé en ce que** l'oxyde métallique du groupe IVB est la zircone.

5. Support catalytique selon l'une quelconque des revendications précédentes, **caractérisé en ce que** l'oxyde métallique du groupe IVB se trouve sous une forme cristalline de type tétragonal.

6. Support catalytique selon l'une quelconque des revendications précédentes, **caractérisé en ce que** sa surface spécifique est supérieure ou égale à 180 $m^2$/g.

7. Support catalytique selon l'une quelconque des revendications précédentes, **caractérisé en ce qu'**il présente un volume de pores supérieur ou égal à 0,25 $cm^3$/g, de préférence compris entre 0,30 et 0,60 $cm^3$/g inclus.

**8.** Support catalytique selon l'une quelconque des revendications précédentes, **caractérisé en ce que** son rayon de pores moyen est supérieur ou égal à 20 Å, de préférence compris entre 20 et 120 Å inclus.

**9.** Support catalytique selon l'une quelconque des revendications précédentes, **caractérisé en ce qu'**il comprend également une quantité variable d'un autre oxyde métallique réfractaire usuellement employé dans l'industrie.

**10.** Support catalytique selon l'une quelconque des revendications précédentes, **caractérisé en ce qu'**il comprend un agent liant constitué d'un oxyde minéral réfractaire habituellement employé dans ce but, choisi notamment dans le groupe constitué par les alumines, les silices, les silice-alumines, les alumino-silicates, les argiles et les mélanges de ces composés.

**11.** Catalyseur supporté, **caractérisé en ce qu'**il comprend un ou plusieurs composés constituant la phase active, déposés sur un support selon l'une quelconque des revendications 1 à 10.

**12.** Catalyseur selon la revendication 11, **caractérisé en ce** ladite phase active comprend au moins un métal ou un composé d'un métal du groupe VIII de la Classification Périodique des Eléments, éventuellement associé à un ou plusieurs composés supplémentaires, métalliques ou non, choisis pour leur activité spécifique dans la réaction à catalyser.

**13.** Catalyseur selon l'une quelconque des revendications 11 et 12, **caractérisé en ce** ladite phase active est constituée d'un ou plusieurs métaux ou composés de métaux du groupe VIII de la Classification Périodique des Eléments, tels que le nickel, le platine, le palladium.

**14.** Catalyseur selon l'une quelconque des revendications 11 et 12, **caractérisé en ce que** ladite phase active est constituée d'au moins un métal du groupe VIII de la Classification Périodique des Eléments, associé à au moins un composé de type acide.

**15.** Catalyseur selon la revendication précédente, **caractérisé en ce que** le métal du groupe VIII est choisi parmi le platine, le palladium, le ruthénium, le rhodium, l'osmium, l'iridium, et le nickel, et le composé de type acide est choisi parmi les ions sulfate, les ions tungstate.

**16.** Catalyseur selon l'une quelconque des revendications 11 et 12, **caractérisé en ce que** la phase active comprend au moins un métal ou un composé d'un métal du groupe VIII de la Classification Périodique des Eléments, associé à au moins un métal ou un composé d'un métal du groupe VIB de la Classification Périodique des Eléments.

**17.** Catalyseur selon la revendication 16, **caractérisé en ce que** le métal du groupe VIII est le cobalt et/ou le nickel et **en ce que** le catalyseur comprend de 2 à 10 % en poids d'oxyde métallique correspondant.

**18.** Catalyseur selon l'une quelconque des revendications 16 et 17, **caractérisé en ce que** le métal du groupe VIB est le molybdène et/ou le tungstène et **en ce que** le catalyseur comprend de 10 à 25 % en poids d'oxyde métallique correspondant.

**19.** Catalyseur selon l'une quelconque des revendications 16 à 18, **caractérisé en ce que** les deux types de métaux sont déposés sur le support sous forme d'oxydes de métaux, lesquels sont ensuite transformés, totalement ou partiellement, en sulfures correspondants (sulfures de métaux) au cours d'une étape d'activation précédant l'utilisation du catalyseur.

**20.** Procédé de préparation d'un support catalytique selon l'une quelconque des revendications 1 à 10, **caractérisé en ce qu'**il comprend essentiellement les étapes suivantes :

a) à partir d'une solution d'au moins un sel d'au moins un métal du groupe IVB, précipitation du ou des oxydes métalliques correspondants,
b) mûrissement à reflux du précipité obtenu, en portant à reflux la solution dans laquelle est précipité le gel d'oxyde métallique du groupe IVB pendant une durée comprise entre 1 et 50 heures,
c) addition de silice en une quantité telle que le rapport entre la quantité d'oxyde métallique du groupe IVB et la quantité de silice contenues dans le support final est compris entre 5 et 70,
d) mûrissement du mélange, sous agitation, en maintenant une agitation vigoureuse du mélange des gels de silice et d'oxyde métallique du groupe IVB, pendant une durée avantageusement comprise entre 1 et 100 heures,

e) lavage, filtration puis séchage du solide obtenu,
f) mise en forme du solide,
g) calcination du solide.

**21.** Procédé selon la revendication précédente, **caractérisé en ce que** l'étape (a) de précipitation du ou des oxyde(s) métallique(s) du groupe IVB est effectuée par addition d'une solution basique à une solution dans laquelle sont dissous des sels métalliques appropriés.

**22.** Procédé selon l'une quelconque des revendications 20 et 21, **caractérisé en ce que** l'étape (b) de mûrissement s'effectue en portant à reflux la solution dans laquelle est précipité le gel d'oxyde métallique du groupe IVB pendant une durée comprise entre 12 et 36 heures.

**23.** Procédé selon l'une quelconque des revendications 20 à 23, **caractérisé en ce que** l'étape (g) de calcination a lieu à une température comprise entre 550 et 850 °C pendant une durée qui est de préférence comprise entre 2 et 6 heures.

**24.** Procédé de préparation d'un catalyseur selon l'une quelconque des revendications 11 à 19, **caractérisé en ce qu'**il comprend le dépôt d'au moins une phase active sur un support selon l'une quelconque des revendications 1 à 10.

**25.** Procédé de préparation d'un catalyseur selon l'une quelconque des revendications 11 à 19, **caractérisé en ce qu'**il comprend les étapes suivantes :

a) à partir d'une solution d'au moins un sel d'au moins un métal du groupe IVB, précipitation du ou des oxydes métalliques correspondants,
b) mûrissement à reflux du précipité obtenu, en portant à reflux la solution dans laquelle est précipité le gel d'oxyde métallique du groupe IVB pendant une durée comprise entre 1 et 50 heures,
c) addition de silice en une quantité telle que le rapport entre la quantité d'oxyde métallique du groupe IVB et la quantité de silice contenues dans le support final est compris entre 5 et 70,
d) mûrissement du mélange, sous agitation, en maintenant une agitation vigoureuse du mélange des gels de silice et d'oxyde métallique du groupe IVB, pendant une durée avantageusement comprise entre 1 et 100 heures,
e) lavage, filtration puis séchage du solide obtenu,
f) mise en forme du solide,
g) calcination du solide.

au moins une étape de dépôt de phase active étant insérée, entre les étapes e) et f) ou entre les étapes f) et g).

**26.** Utilisation d'un catalyseur selon l'une quelconque des revendications 11 à 19 dans un procédé de traitement de coupes hydrocarbonées.

**27.** Utilisation d'un catalyseur selon la revendication 13 dans une réaction d'hydrogénation d'hydrocarbures.

**28.** Utilisation d'un catalyseur selon les revendications 14 et 15 dans une réaction nécessitant l'emploi d'un catalyseur de type acide ou superacide, telles que notamment des réactions d'isomérisation de paraffines ou d'oléfines, des réactions de décyclisation d'hydrocarbures naphténiques, des réactions d'alkylation, des réactions d'oligomérisation, des réactions de déshydratation d'hydrocarbures, des réactions d'hydrogénation de coupes pétrolières.

**29.** Utilisation d'un catalyseur selon les revendications 14 et 15, dans des réactions d'hydrodéaromatisation et/ou de désulfuration profonde de coupes pétrolières, dont notamment des gazoles.

**30.** Utilisation d'un catalyseur selon les revendications 14 et 15, dans une réaction d'hydrocraquage et/ou d'hydroisomérisation de paraffines longues, à plus de 7 atomes de carbone.

**31.** Utilisation d'un catalyseur selon l'une quelconque des revendications 16 à 19 dans un procédé d'hydrotraitement tel que notamment un procédé d'hydrodésulfuration, d'hydrodéazotation, d'hydrodémétallation ou d'hydrogénation de coupes pétrolières telles que des essences, des gazoles, des distillats sous vide, des résidus atmosphériques ou des résidus sous vide.

**EP 1 053 785 B1**

**Claims**

1. A catalytic support essentially composed of at least one metal oxide of group IVB of the Periodic Classification of the elements, in which silica is incorporated, **characterised in that** the mass ratio between the quantity of metal oxide of group IVB and the quantity of silica contained therein is between 5 and 70, **in that** the metal oxide of group IVB is in crystalline form, and **in that** the specific surface of the support is higher than or equal to 160 m$^2$/g.

2. A catalytic support according to claim 1, **characterised in that** the mass ratio between the metal oxide of group IVB and silica is between 8 and 30.

3. A catalytic support according to any one of the preceding claims, **characterised in that** the metal oxide of group IVB is chosen from zirconium oxide, titanium oxide or a mixture of these two oxides.

4. A catalytic support according to claim 3, **characterised in that** the metal oxide of group IVB is zirconium oxide.

5. A catalytic support according to any one of the preceding claim, **characterised in that** the metal oxide of group IVB is present in a crystalline form of tetragonal type.

6. A catalytic support according to any one of the preceding claims, **characterised in that** its specific surface is higher than or equal to 180 m$^2$/g.

7. A catalytic support according to any one of the preceding claims, **characterised in that** it has a pore volume higher than or equal to 0.25 cm$^3$/g, preferably between 0.30 and 0.60 cm$^3$/g inclusive.

8. A catalytic support according to any one of the preceding claims, **characterised in that** its mean pore radius is higher than or equal to 20 Å, preferably between 20 and 120 Å inclusive.

9. A catalytic support according to any one of the preceding claims, **characterised in that** it also comprises a variable quantity of another refractory metal oxide commonly used in the industry.

10. A catalytic support according to any one of the preceding claims, **characterised in that** it comprises a binder formed by a refractory mineral oxide commonly used for this purpose, chosen in particular from the group comprising the aluminas, the silicas, the silico-aluminas, aluminosilicates, the clays and mixtures of these compounds.

11. A supported catalyst, **characterised in that** it comprises one or more compounds constituting the active phase, deposited on a support according to any one of claims 1 to 10.

12. A catalyst according to claim 11, **characterised in that** said active phase comprises at least one metal or a compound of a metal of group VIII of the Periodic Classification of the elements, optionally associated with one or more supplementary metallic or non-metallic compounds, chosen for their specific activity in the reaction to be catalysed.

13. A catalyst according to either one of claims 11 and 12, **characterised in that** said active phase comprises one or more metals or compounds of metals of group VIII of the Periodic Classification of the elements, such as nickel, platinum, palladium.

14. A catalyst according to either one of claims 11 and 12, **characterised in that** said active phase comprises at least one metal of group VIII of the Periodic Classification of the elements, associated with at least one compound of acid type.

15. A catalyst according to the preceding claim, **characterised in that** the metal of group VIII is chosen from platinum, palladium, ruthenium, rhodium, osmium, iridium and nickel, and the compound of acid type is chosen from the sulphate ions, the tungstate ions.

16. A catalyst according to either one of claims 11 and 12, **characterised in that** the active phase comprises at least one metal or a compound of a metal of group VIII of the Periodic Classification of the elements, associated with at least one metal or a compound of a metal of group VIB of the Periodic Classification of the elements.

17. A catalyst according to claim 16, **characterised in that** the metal of group VIII is cobalt and/or nickel, and **in that** the catalyst comprises 2 to 10 % by weight of corresponding metal oxide.

18. A catalyst according to either one of claims 16 and 17, **characterised in that** the metal of group VIB is molybdenum and/or tungsten, and **in that** the catalyst comprises 10 to 25 % by weight of corresponding metal oxide.

19. A catalyst according to any of claims 16 to 18, **characterised in that** the two types of metals are deposited on the support in the form of metal oxides which are then transformed, wholly or partly, into corresponding sulphides (metal sulphides) in the course of an activation step preceding the use of the catalyst.

20. A method of preparing a catalytic support according to any one of claims 1 to 10, **characterised in that** it comprises the following steps:

> a) starting from a solution of at least one salt of at least one metal of group IVB, precipitation of the corresponding metal oxide or oxides,
> b) maturing under reflux the precipitate obtained, by bringing to reflux the solution in which is precipitated the metal oxide gel of group IVB for a period of between 1 and 50 hours,
> c) addition of silica in a quantity such that the ratio between the quantity of metal oxide of group IVB and the quantity of silica contained in the end support is between 5 and 70,
> d) maturing the mixture, with agitation, by maintaining vigorous agitation of the mixture of gels of silica and metal oxide of group IVB, for a period advantageously of between 1 and 100 hours,
> e) washing, filtering and then drying the solid obtained,
> f) moulding the solid,
> g) calcining the solid.

21. A method according to the preceding claim, **characterised in that** the step (a) of precipitating the metal oxide or oxides of group IVB is carried out by adding a basic solution to a solution in which suitable metal salts are dissolved.

22. A method according to either one of claims 20 and 21, **characterised in that** the maturing step (b) is carried out by bringing to reflux the solution in which is precipitated the metal oxide gel of group IVB for a period of between 12 and 36 hours.

23. A method according to any one of claims 20 to 23 [sic], **characterised in that** the calcination step (g) takes place at a temperature of between 550 and 850°C for a period which is preferably between 2 and 6 hours.

24. A method of preparing a catalyst according to any one of claims 11 to 19, **characterised in that** it comprises depositing at least one active phase on a support according to any one of claims 1 to 10.

25. A method of preparing a catalyst according to any one of claims 11 to 19, **characterised in that** it comprises the following steps:

> a) starting from a solution of at least one salt of at least one metal of group IVB, precipitation of the corresponding metal oxide or oxides,
> b) maturing under reflux the precipitate obtained, by bringing to reflux the solution in which is precipitated the metal oxide gel of group IVB for a period of between 1 and 50 hours,
> c) addition of silica in a quantity such that the ratio between the quantity of metal oxide of group IVB and the quantity of silica contained in the end support is between 5 and 70,
> d) maturing the mixture, with agitation, by maintaining vigorous agitation of the mixture of gels of silica and metal oxide of group IVB, for a period advantageously of between 1 and 100 hours,
> e) washing, filtering and then drying the solid obtained,
> f) moulding the solid,
> g) calcining the solid,

at least step of depositing the active phase being inserted between steps e) and f) or between steps f) and g).

26. Use of a catalyst according to any one of claims 11 and 19 in the method for treating hydrocarbon fractions.

27. Use of a catalyst according of a catalyst according to claim 13 in a reaction for the hydrogenation of hydrocarbons.

28. Use of a catalyst according of a catalyst according to claims 14 and 15 in a reaction necessitating the use of a catalyst of the acid or superacid type, in particular such as reactions for the isomerisation of paraffins or olefins, reactions for the decyclisation of naphthenic hydrocarbons, alkylation reactions, oligomerisation reactions, reactions for the dehydration of hydrocarbons, reactions for the hydrogenation of petroleum fractions.

29. Use of a catalyst according of a catalyst according to claims 14 and 15 in reactions for the hydro-dearomatisation and/or deep desulphurisation of petroleum fractions, including in particular the gas oils.

30. Use of a catalyst according of a catalyst according to claims 14 and 15 in a reaction for the hydrocracking and/or hydroisomerisation of long paraffins, having more than 7 carbon atoms.

31. Use of a catalyst according to any one of claims 16 to 19 in a hydrotreatment process, in particular such as a process for the hydrodesulphurisation, hydrodenitrification, dehydrodemetallisation or hydrogenation of petroleum fractions, such as gasolines, gas oils, vacuum distillates, atmospheric residues or vacuum residues.

**Patentansprüche**

1. Katalytischer Träger, der im Wesentlichen aus mindestens dem Oxid eines Metalls der Gruppe IVB des Periodensystems der Elemente aufgebaut ist, in welches Siliziumdioxid eingebaut ist, **dadurch gekennzeichnet, dass** das Masseverhältnis zwischen der Menge des Oxids des Metalls der Gruppe IVB und der Menge des Siliziumdioxids, die es enthält, zwischen 5 und 70 liegt, dass das Oxid des Metalls der Gruppe IVB in kristalliner Form vorliegt, und dass die spezifische Oberfläche des Trägers größer oder gleich 160 $m^2$/g ist.

2. Katalytischer Träger gemäß Anspruch 1, **dadurch gekennzeichnet, dass** das Masseverhältnis zwischen dem Oxid des Metalls der Gruppe IVB und dem Siliziumdioxid zwischen 8 und 30 liegt.

3. Katalytischer Träger gemäß irgendeinem der vorangehenden Ansprüche, **dadurch gekennzeichnet, dass** das, Oxid des Metalls der Gruppe IVB unter Zirkonoxid, dem Oxid von Titan oder einer Mischung dieser beiden Oxide ausgewählt ist.

4. Katalytischer Träger gemäß Anspruch 3, **dadurch gekennzeichnet, dass** das Oxid des Metalls der Gruppe IVB Zirkonoxid ist.

5. Katalytischer Träger gemäß irgendeinem der vorangehenden Ansprüche, **dadurch gekennzeichnet, dass** das Oxid des Metalls der Gruppe IVB sich in der kristallinen Form des tetragonalen Typs befindet.

6. Katalytischer Träger gemäß irgendeinem der vorangehenden Ansprüche, **dadurch gekennzeichnet, dass** die spezifische Oberfläche größer oder gleich 180 $m^2$/g beträgt.

7. Katalytischer Träger gemäß irgendeinem der vorangehenden Ansprüche, **dadurch gekennzeichnet, dass** er ein Porenvolumen von größer oder gleich 0,25 $cm^3$/g, vorzugsweise zwischen einschließlich 0,30 und 0,60 $cm^3$/g, zeigt.

8. Katalytischer Träger gemäß irgendeinem der vorangehenden Ansprüche, **dadurch gekennzeichnet, dass** sein mittlerer Porenradius größer oder gleich 20 Å, vorzugsweise zwischen einschließlich 20 und 120 Å, beträgt.

9. Katalytischer Träger gemäß irgendeinem der vorangehenden Ansprüche, **dadurch gekennzeichnet, dass** er ferner eine variable Menge eines anderen, hitzebeständigen Metalloxids umfasst, das gewöhnlich in der Industrie verwendet wird.

10. Katalytischer Träger gemäß irgendeinem der vorangehenden Ansprüche, **dadurch gekennzeichnet, dass** er ein Bindemittel umfasst, das aus einem hitzebeständigen, mineralischen Oxid aufgebaut ist, welches gewöhnlich zu diesem Zweck angewandt wird, ausgewählt insbesondere aus der Gruppe, die aus Tonerden, Siliziumdioxiden, Siliziumdioxid-Tonerden, Aluminiumsilikaten, Tonen sowie Mischungen dieser Verbindungen besteht.

11. Getragener Katalysator, **dadurch gekennzeichnet, dass** er ein oder mehrere, die aktive Phase aufbauende Verbindungen umfasst, die auf einem Träger gemäß irgendeinem der vorangehenden Ansprüche 1 bis 10 abgeschieden sind.

12. Katalysator gemäß Anspruch 11, **dadurch gekennzeichnet, dass** die aktive Phase mindestens ein Metall oder eine Verbindung eines Metalls der Gruppe VIII des Periodensystems der Elemente umfasst, gegebenenfalls verbunden mit einem oder mehreren, metallischen oder nicht-metallischen Zusatzverbindungen, die nach deren spezifischer Aktivität auf die zu katalysierenden Reaktionen ausgewählt sind.

13. Katalysator gemäß irgendeinem der Ansprüche 11 und 12, **dadurch gekennzeichnet, dass** die aktive Phase aus einem oder mehreren Metallen oder Verbindungen von Metallen der Gruppe VIII des Periodensystems der Elemente aufgebaut ist, wie Nickel, Platin oder Palladium.

14. Katalysator gemäß irgendeinem der Ansprüche 11 und 12, **dadurch gekennzeichnet, dass** die aktive Phase aus mindestens einem Metall der Gruppe VIII des Periodensystems der Elemente aufgebaut ist, assoziiert mit mindestens einer Säureverbindung.

15. Katalysator gemäß dem vorangehenden Anspruch, **dadurch gekennzeichnet, dass** das Metall der Gruppe VIII ausgewählt ist unter Platin, Palladium, Ruthenium, Rhodium, Osmium, Iridium und Nickel, und dass die Säureverbindung ausgewählt ist unter den Sulfationen und den Wolframionen.

16. Katalysator gemäß irgendeinem der Ansprüche 11 und 12, **dadurch gekennzeichnet, dass** die aktive Phase mindestens ein Metall oder eine Verbindung eines Metalls der Gruppe VIII des Periodensystems der Elemente umfasst, verbunden mit mindestens einem Metall oder einer Verbindung eines Metalls der Gruppe VIB des Periodensystems der Elemente.

17. Katalysator gemäß Anspruch 16, **dadurch gekennzeichnet, dass** das Metall der Gruppe VIII Kobalt und/oder Nickel ist, und dass der Katalysator 2 bis 10 Gew.-% des entsprechenden Metalloxids umfasst.

18. Katalysator gemäß einem der Ansprüche 16 und 17, **dadurch gekennzeichnet, dass** das Metall der Gruppe VIB Molybdän und/oder Wolfram ist, und dass der Katalysator 10 bis 25 Gew.-% des entsprechenden Metalloxids umfasst.

19. Katalysator gemäß irgendeinem der Ansprüche 16 bis 18, **dadurch gekennzeichnet, dass** die zwei Arten der Metalle auf dem Träger in Form der Metalloxide abgeschieden sind, welche dann im Verlauf eines der Anwendung des Katalysators vorangehenden Aktivierungsschritts vollständig oder teilweise in entsprechende Sulfide (Metallsulfide) umgewandelt werden.

20. Verfahren zur Herstellung eines katalytischen Trägers gemäß irgendeinem der Ansprüche 1 bis 10, **dadurch gekennzeichnet, dass** es im Wesentlichen folgende Schritte umfasst:

    a) ausgehend von einer Lösung von mindestens einem Salz mindestens eines Metalls der Gruppe IVB, Fällung des oder der entsprechenden Metalloxide,
    b) Reifung des erhaltenden Präzipitats unter Rückfluss, indem die Lösung, aus der das Oxidgel des Metalls der Gruppe IVB gefällt wurde, während einer Dauer von 1 bis 50 Stunden unter Rückfluss gesetzt wird,
    c) Zugabe von Siliziumdioxid in einer Menge derart, dass das Verhältnis zwischen der Menge des Oxids des Metalls der Gruppe IVB und der Menge des Siliziumdioxids, die im fertigen Träger enthalten ist, zwischen 5 und 70 liegt,
    d) Reifung der Mischung unter Agitation, wobei eine heftige Agitation der Mischung der Gele des Siliziumdioxids und des Oxids des Metalls der Gruppe IVB aufrechterhalten wird, während einer Dauer von vorteilhafterweise 1 bis 100 Stunden,
    e) Waschung, Filtrierung und dann Trocknung des erhaltenen Feststoffs,
    f) Bringen in feste Form,
    g) Kalzinierung des Feststoffs.

21. Verfahren gemäß dem vorangehenden Anspruch, **dadurch gekennzeichnet, dass** der Schritt a) der Fällung des oder der Oxids (Oxide) des Metalls (der Metalle) der Gruppe IVB durch Zugabe einer basischen Lösung zu einer Lösung, in der die geeigneten Metallsalze aufgelöst sind, bewirkt wird.

22. Verfahren gemäß irgendeinem der Ansprüche 20 und 21, **dadurch gekennzeichnet, dass** der Schritt b) der Reifung bewirkt wird, indem die Lösung, in der das Gel des Oxids des Metalls der Gruppe IVB präzipitiert ist, während einer Dauer von 12 bis 36 Stunden unter Rückfluss gesetzt wird.

**23.** Verfahren gemäß irgendeinem der Ansprüche 20 bis 23, **dadurch gekennzeichnet, dass** der Schritt g) der Kalzinierung bei einer Temperatur von 550 bis 800 °C während einer Dauer stattfindet, die vorzugsweise zwischen 2 und 6 Stunden liegt.

**24.** Verfahren zur Herstellung eines Katalysators gemäß irgendeinem der Ansprüche 11 bis 19, **dadurch gekennzeichnet, dass** es den Niederschlag mindestens einer aktiven Phase auf einem Träger gemäß irgendeinem der Ansprüche 1 bis 10 umfasst.

**25.** Verfahren zur Herstellung eines Katalysators gemäß irgendeinem der Ansprüche 11 bis 19, **dadurch gekennzeichnet, dass** es die folgenden Schritte umfasst:

a) ausgehend von einer Lösung von mindestens einem Salz mindestens eines Metalls der Gruppe IVB, Fällung des oder der entsprechenden Metalloxide,
b) Reifung des erhaltenen Präzipitats unter Rückfluss, indem die Lösung, aus der das Oxidgel des Metalls der Gruppe IVB gefällt wurde, während einer Dauer von 1 bis 50 Stunden unter Rückfluss gesetzt wird,
c) Zugabe von Siliziumdioxid in einer Menge derart, dass das Verhältnis zwischen der Menge des Oxids des Metalls der Gruppe IVB und der Menge des Siliziumdioxids, die im fertigen Träger enthalten ist, zwischen 5 und 70 liegt,
d) Reifung der Mischung unter Agitation, wobei eine heftige Agitation der Mischung der Gele des Siliziumdioxids und des Oxids des Metalls der Gruppe IVB aufrechterhalten wird, während einer Dauer von vorteilhafterweise zwischen 1 und 100 Stunden,
e) Waschung, Filterung und dann Trocknung des erhaltenen Feststoffs,
f) Bringen in feste Form,
g) Kalzinierung des Feststoffs,

wobei mindestens ein Schritt der Ablagerung der aktiven Phase zwischen den Schritten e) und f) oder zwischen den Schritten f) und g) eingefügt wird.

**26.** Verwendung eines Katalysators gemäß irgendeinem der Ansprüche 11 bis 19 in einem Verfahren zur Behandlung von Kohlenwasserstoffverschnitten.

**27.** Verwendung eines Katalysators gemäß Anspruch 13 in einer Hydrierreaktion von Kohlenwasserstoffen.

**28.** Verwendung eines Katalysators gemäß den Ansprüchen 14 und 15 in einer Reaktion, die die Verwendung eines Katalysators vom Säure- oder Supersäure-Typ erfordert, wie insbesondere Isomerisierungsreaktionen von Paraffinen oder Olefinen, Dezyklisierungs-Reaktionen von Naphten-Kohlenwasserstoffen, Alkylierungsreaktionen, Oligomerisierungsreaktionen, Dehydratationsreaktionen von Kohlenwasserstoffen, oder Dehydrierreaktionen von Erdölverschnitten.

**29.** Verwendung eines Katalysators gemäß den Ansprüchen 14 und 15 in Hydrodearomatisierungs- und/oder starken Entschwefelungsreaktionen von Erdölverschnitten, und von diesen insbesondere von Dieselöl.

**30.** Verwendung eines Katalysators gemäß den Ansprüchen 14 und 15 in einer Hydrocrack- und/oder Hydroisomerisierungsreaktion von langen Paraffinen mit mehr als 7 Kohlenstoffatomen.

**31.** Verwendung eines Katalysators gemäß irgendeinem der Ansprüche 16 bis 19, in einem Hydrobehandlungsverfahren, wie insbesondere einem Hydroentschwefelungs-, einem Hydrodeazotier-, einem Hydrodemetallisations- oder HydrierVerfahren von Erdölverschnitten, wie Benzinkraftstoffen, Dieselkraftstoffen, Vakuumdestillaten, atmosphärischen Resten oder Vakuumresten.

**EP 1 053 785 B1**

**RÉFÉRENCES CITÉES DANS LA DESCRIPTION**

**Documents brevets cités dans la description**

- US 3686095 A
- US 5021385 A
- FR 2661171
- US 5262373 A
- FR 2709432
- WO 9718892 A